Europäisches Patentamt

European Patent Office

Office européen des brevets

(19)

(11) Veröffentlichungsnummer : **0 070 395**
**B1**

(12) **EUROPÄISCHE PATENTSCHRIFT**

(45) Veröffentlichungstag der Patentschrift :
10.09.86

(51) Int. Cl.⁴ : **C 07 D 213/85**

(21) Anmeldenummer : **82105359.2**

(22) Anmeldetag : **18.06.82**

(54) **Verfahren zur Gewinnung von 3-Cyanpyridin.**

(30) Priorität : **22.07.81 DE 3128956**

(43) Veröffentlichungstag der Anmeldung :
**26.01.83 Patentblatt 83/04**

(45) Bekanntmachung des Hinweises auf die Patenterteilung : **10.09.86 Patentblatt 86/37**

(84) Benannte Vertragsstaaten :
**AT BE CH DE FR GB IT LI NL SE**

(56) Entgegenhaltungen :
**DE-A- 2 039 497**
**DE-A- 2 435 344**
**DE-C- 862 011**
**GB-A- 777 746**
**US-A- 2 861 999**

(73) Patentinhaber : **Degussa Aktiengesellschaft**
**Weissfrauenstrasse 9**
**D-6000 Frankfurt am Main 1 (DE)**

(72) Erfinder : **Beschke, Helmut**
**Greifenhagenstrasse 23**
**D-6450 Hanau 9 (DE)**
Erfinder : **Dahm, Franz Ludwig, Dipl.-Ing.**
**Kurmainzer Strasse 4**
**D-8755 Alzenau (DE)**
Erfinder : **Friedrich, Heinz, Dr. Dipl.-Chem.**
**Grünaustrasse 4**
**D-6450 Hanau 9 (DE)**

EP 0 070 395 B1

## Beschreibung

Die Erfindung betrifft ein Verfahren zur Gewinnung von 3-Cyanpyridin aus den bei der katalytischen Umsetzung von 3-Methylpyridin mit Ammoniak und Sauerstoff anfallenden Gasgemischen.

Es sind zahlreiche Verfahren für die Herstellung von 3-Cyanpyridin durch katalytische Umsetzung von 3-Methylpyridin mit Ammoniak und Sauerstoff in der Gasphase bekannt. Diese Verfahren unterscheiden sich im wesentlichen in den zur Anwendung gelangenden Katalysatoren. Beispielsweise werden als Katalysatoren Zinnvanadat im Gemisch mit Diphosphorpentoxid auf Aluminiumoxid, Silikagel oder deren Gemischen (JA-AS 42-6066) oder Molybdänoxid im Gemisch mit Oxiden des Vanadins, Chroms, Mangans oder Kobalts auf Alumiumoxid, Magnesiumoxid, Siliciumoxid oder Titanoxid (JP-AS 45-13572) oder reines Divanadinpentoxid bestimmter Oberfläche und Korngröße (DE-OS 2 435 344) eingesetzt. Besonders geeignet sind Katalysatoren, die dadurch hergestellt werden, daß Mischungen, die Antimon und Vanadin und wenigstens eines der Elemente Titan, Eisen, Kupfer, Kobalt, Mangan und Nickel und gegebenenfalls eine Trägersubstanz enthalten, durch Erhitzen auf Temperaturen von 600 bis 1 100 °C in Gegenwart von Sauerstoff vorbehandelt werden (DE-PS 2 039 497).

Die bei der katalytischen Umsetzung entstehenden Gasgemische enthalten im allgemeinen — unabhängig davon, welcher Katalysator verwendet wird — 3-Cyanpyridin, Ammoniak, gegebenenfalls nicht umgesetztes 3-Methylpyridin und Nebenprodukte, wie Wasser, Kohlendioxid, Nicotinsäureamid und Cyanwasserstoff. Die Aufarbeitung ist im wesentlichen auf die Gewinnung des 3-Cyanpyridins sowie auf die Rückgewinnung des überschüssigen Ammoniaks und des nicht umgesetzten 3-Methylpyridins gerichtet. Damit das Ammoniak im Kreislauf geführt und erneut verwendet werden kann, ist erforderlich, es von Kohlendioxid und von den übrigen Nebenprodukten zu trennen.

Bei einem bekannten Verfahren zur Herstellung von 3-Cyanpyridin durch katalytische Umsetzung von 3-Methylpyridin wird das Umsetzungsgemisch mit Trockeneis gekühlt, die hierbei kondensierten Anteile werden mit einem Lösungsmittel, wie Benzol, gewaschen, und aus dieser Lösung wird das 3-Cyanpyridin und gegebenenfalls vorhandenes 3-Methylpyridin durch Destillation gewonnen (US-PS 2 861 999). Bei einem anderen Verfahren wird das Umsetzungsgemisch mit Wasser gewaschen, die wäßrige Lösung wird mit Äther extrahiert, und durch Abdestillieren des Äthers wird das 3-Cyanpyridin gewonnen (GB-PS 777 746). Über die Rückgewinnung des überschüssigen Ammoniaks — es fällt in beiden Fällen als wäßrige Lösung an — wird nichts ausgesagt. Die Rückgewinnung würde aufwendig und nicht ohne erhebliche Verluste möglich sein.

Bei einem weiteren Verfahren wird das Umsetzungsgemisch bei niedriger Temperatur mit Methanol gewaschen und auf diese Weise das 3-Cyanpyridin, gegebenenfalls vorhandenes 3-Methylpyridin und etwas Ammoniak abgetrennt (DE-OS 2 435 344). Bei diesem Verfahren verbleiben die Hauptanteile Ammoniak im Restgas. Nachteilig ist aber, daß dieses auch Nebenprodukte, wie insbesondere Kohlendioxid, enthält, die sich anreichern und die Umsetzung stören, wenn das Restgas wiederholt im Kreislauf geführt wird.

Es ist nun ein Verfahren zur Gewinnung von 3-Cyanpyridin aus den bei der katalytischen Umsetzung von 3-Methylpyridin mit Ammoniak und Sauerstoff anfallenden Gasgemischen durch deren Behandlung mit Wasser gefunden worden, das dadurch gekennzeichnet ist, daß die Gasgemische zunächst bei Temperaturen von 30 bis 60 °C und dann bei um etwa 10 bis 30 °C niedrigeren Temperaturen behandelt werden. Auf diese Weise gelingt es, nicht nur reines 3-Cyanpyridin zu gewinnen, sondern auch das Ammoniak und gegebenenfalls vorhandenes 3-Methylpyridin so abzuscheiden und zurückzugewinnen, daß diese Substanzen unmittelbar und ohne Verluste im Kreislauf zurückgeführt werden können.

Nach dem erfindungsgemäßen Verfahren kann das 3-Cyanpyridin aus allen Gasgemischen gewonnen werden, die bei den üblichen katalytischen Umsetzungen von 3-Methylpyridin mit Ammoniak und Sauerstoff in der Gasphase anfallen, insbesondere aus den Gasgemischen, die bei der Umsetzung unter Verwendung der Katalysatoren nach der DE-OS 2 039 497 oder der DE-OS 3 107 755 entstehen.

Zur Durchführung des erfindungsgemäßen Verfahrens werden die warmen Gasgemische zweckmäßigerweise unmittelbar nach Austritt aus dem Reaktor in Gegenwart von Wasser in einer ersten Stufe auf Temperaturen etwa von 30 bis 60 °C, vorzugsweise auf Temperaturen von 35 bis 55 °C, und in einer zweiten Stufe auf um etwa 10 bis 30 °C, vorzugsweise um 15 bis 25 °C, niedrigere Temperaturen gebracht. Der Druck kann hierbei weitgehend beliebig gewählt werden, jedoch empfiehlt es sich, bei Normaldruck oder nur mäßig erniedrigten oder erhöhten Drücken zu arbeiten. Solche Drücke treten gegebenenfalls auf, indem die Gase durch die Anlage gesaugt oder gedrückt werden.

Vorzugsweise werden die Gasgemische in der ersten wie in der zweiten Stufe auf die genannten Temperaturen gebracht, indem sie mit wäßrigen Waschflüssigkeiten gewaschen werden. Enthalten die aus dem Reaktor austretenden Gasgemische jedoch Wasserdampf in Mengen, die zur Bildung einer wäßrigen Lösung ausreichen, in der das 3-Cyanpyridin, gegebenenfalls vorhandenes 3-Methylpyridin, sowie das Kohlendioxid und andere Nebenprodukte aufgenommen werden können, bedarf es in der ersten Stufe lediglich einer Abkühlung der Gasgemische.

Wenngleich als Waschflüssigkeit in der ersten Stufe reines Wasser verwendbar ist, ist es im allgemeinen zweckmäßig, Wasser einzusetzen, das Ammoniak enthält. In welcher Menge und mit welchem Gehalt an Ammoniak die Waschflüssigkeit angewendet wird, richtet sich gegebenenfalls nach der Zusammensetzung der zu behandelnden Gasgemische, vornehmlich nach deren Gehalt an Wasser, Ammoniak und Kohlendioxid. In den meisten Fällen, insbesondere bei einer kontinuierlichen Arbeitsweise, bei der die Waschflüssigkeit im Kreislauf geführt wird, ist es zweckmäßig, als Waschflüssigkeit Wasser zu verwenden, das bei der betreffenden Temperatur weitgehend oder vollständig mit Ammoniak gesättigt ist. Vorteilhaft ist es, die Waschflüssigkeit so zu bemessen, daß je Mol Kohlendioxid in den zu behandelnden Gasen mindestens etwa 3 Mol, vorzugsweise mindestens etwa 5 Mol, Ammoniak und je Mol Ammoniak etwa 0,1 bis 0,5 Liter Wasser vorhanden sind. In der zweiten Stufe wird als Waschflüssigkeit Wasser eingesetzt, das von Ammoniak frei ist oder allenfalls einen geringen Gehalt an Ammoniak aufweist. Die Waschflüssigkeit wird zweckmäßigerweise so bemessen, daß je Mol Ammoniak in dem in der zweiten Stufe zu behandelnden Gasgemisch mindestens etwa 0,2 Liter Wasser vorhanden sind.

Die in der ersten Stufe anfallende wäßrige Lösung enthält das 3-Cyanpyridin, gegebenenfalls vorhandenes nicht umgesetztes 3-Methylpyridin, Ammoniak, das Kohlendioxid, und zwar dieses als Ammoniumhydrogencarbonat, sowie die übrigen Nebenprodukte, soweit sie in dem Medium löslich sind. Die in der zweiten Stufe anfallende wäßrige Lösung enthält Ammoniak.

Für die Gewinnung des 3-Cyanpyridins und gegebenenfalls des 3-Methylpyridins aus der in der ersten Stufe anfallenden wäßrigen Lösung wird diese mit einem organischen Lösungsmittel extrahiert. Als Lösungsmittel kommen beispielsweise aliphatische chlorierte Kohlenwasserstoffe oder aromatische, gegebenenfalls chlorierte, Kohlenwasserstoffe, vorzugsweise Benzol, in Frage. Der Extrakt wird fraktioniert destilliert. Das hierbei zurückgewonnene Lösungsmittel kann für weitere Extraktionen eingesetzt werden.

Die nach der Extraktion verbleibende wäßrige Lösung enthält Ammoniumhydrogencarbonat neben Ammoniak. Aus dieser Lösung wird das Kohlendioxid desorbiert, indem die Lösung, zweckmäßigerweise unter Aufgabe von Wasser, unter Drücken etwa von 5 bis 12 bar, vorzugsweise von 6 bis 10 bar, bei Temperaturen etwa von 120 bis 170 °C, vorzugsweise von 130 bis 160 °C, behandelt wird. Das Kohlendioxid entweicht mit Anteilen von Wasser. Vorteilhaft ist es im allgemeinen, Kohlendioxid und Wasser in solchem Maße abzutreiben beziehungsweise Wasser in solchem Maße zuzuführen, daß eine Restlösung anfällt, die etwa 2 bis 10 Mol, vorzugsweise 3 bis 8 Mol, Ammoniak je Liter Wasser und höchstens etwa 0,4 Mol, vorzugsweise höchstens 0,3 Mol, Kohlendioxid je Mol Ammoniak enthält. Eine derartige Restlösung ist im allgemeinen unmittelbar für den Einsatz als Waschflüssigkeit in der ersten Stufe geeignet.

Die in der zweiten Stufe anfallende wäßrige Lösung wird zur Gewinnung des Ammoniaks bei Temperaturen etwa von 80 bis 140 °C, vorzugsweise von 90 bis 130 °C, und Drücken etwa von 1,5 bis 3,0 bar, vorzugsweise von 1,8 bis 2,7 bar, desorbiert. Im allgemeinen kann unmittelbar das hierbei anfallende Ammoniak zurückgeführt und für neue Umsetzungen verwendet und das vom Ammoniak befreite Wasser als Waschflüssigkeit in der zweiten Stufe eingesetzt werden.

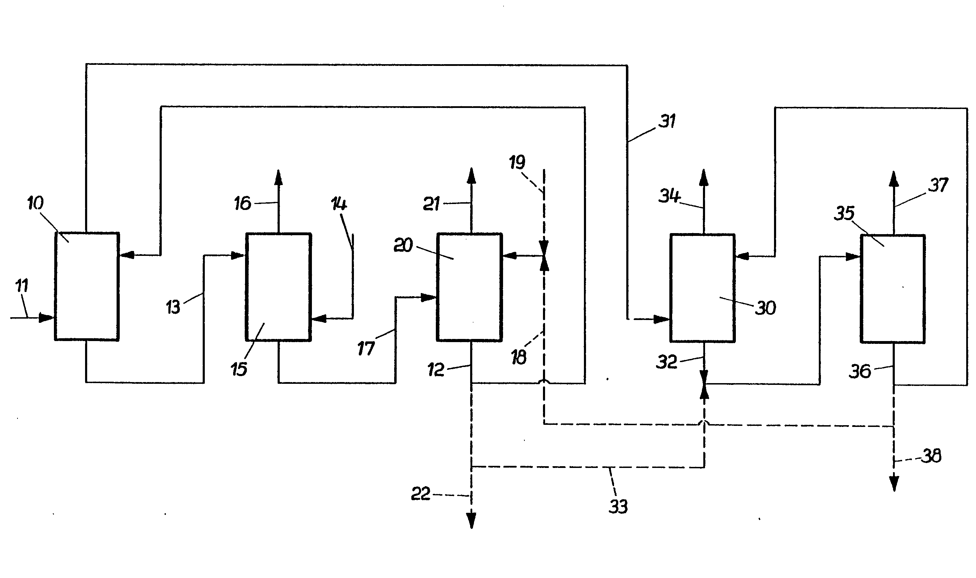

Eine bevorzugte Gestaltung des Verfahrens, die insbesondere für eine kontinuierliche Arbeitsweise geeignet ist, ist die in der Abbildung gezeigte :

Die aus dem Reaktor austretenden Gase (11) werden für die Wäsche in der ersten Stufe in einen Gaswäscher (10) geleitet, der bei 30 bis 60 °C betrieben wird. Auf diesen Wäscher wird als Waschflüssigkeit die Restlösung (12) aus (20) aufgegeben. Die aus dem Wäscher (10) abfließende wäßrige Lösung (13) gelangt in die Extraktion (15). Hier wird durch ein organisches Lösungsmittel (14) das 3-Cyanpyridin und gegebenenfalls vorhandenes 3-Methylpyridin extrahiert. Aus diesem Extrakt (16) wird durch eine Destillation das 3-Cyanpyridin und das 3-Methylpyridin gewonnen. Das hierbei anfallende organische Lösungsmittel (14) wird in die Extraktion zurückgeführt. Verluste an organischem Lösungsmittel werden durch Zugabe von frischem Lösungsmittel ausgeglichen. Aus der nach der Extraktion (15) verbleibenden wäßrigen Lösung (17) wird in der Desorption (20) Kohlendioxid und Wasser (21) abgetrieben. In die Desorption (20) wird Wasser (19) eingeführt. Die wäßrige Restlösung (12) aus (20) wird als Waschflüssigkeit in den Gaswäscher (10) der ersten Stufe aufgegeben.

Das Restgas (31) aus dem Gaswäscher (10) in der ersten Stufe wird dem Gaswäscher (30) der zweiten Stufe zugeführt, der bei einer um 10 bis 30 °C niedrigeren Temperatur als der Gaswäscher (10) der ersten Stufe betrieben wird. Auf den Gaswäscher (30) wird als Waschflüssigkeit das von Ammoniak in der Desorption (35) befreite Wasser (36) aufgegeben. Das nach der Wäsche in (30) verbleibende Restgas (34) wird verworfen. Die aus dem Gaswäscher (30) abfließende wäßrige Ammoniaklösung (32) wird zur Abtreibung des Ammoniaks der Desorption (35) zugeführt. Das abgetriebene Ammoniak (37) wird in die Umsetzung zurückgeführt.

Zur Einstellung und Aufrechterhaltung stationärer Verhältnisse im Falle kontinuierlicher Arbeitsweise kann es zufolge von Schwankungen in der Zusammensetzung und der Temperatur der Reaktionsgase erforderlich sein, einen kleinen Anteil der aus (20) ablaufenden Flüssigkeit (12) abzuzweigen und der aus (30) ablaufenden Flüssigkeit (32) zuzumischen (33) und beziehungsweise oder als Abwasser zu verwerfen (22). Auch kann es erforderlich sein, von der aus (35) ablaufenden Flüssigkeit (36) einen kleinen Anteil abzuzweigen und ganz oder teilweise an Stelle von Wasser (19) in (20) aufzugeben (18) und beziehungsweise oder als Abwasser zu verwerfen (38).

Beispiel

Es wurde eine Einrichtung gemäß Abbildung verwendet. Die Gaswäscher (10) und (30) bestanden aus Füllkörpersäulen. In einem vorgeschalteten Reaktor wurde stündlich ein Gasgemisch aus 4 800 Normalliter Luft, 13,5 kg Wasser, 12,7 kg Ammoniak und 11,0 kg 3-Methylpyridin umgesetzt. Ein Anteil des Gasgemisches von stündlich 8,1 kg Ammoniak und 12,6 kg Wasser war Abgas (37) aus der Desorption (35). Die Umsetzung erfolgte an einem Festbettkatalysator, der nach der DE-OS 3 107 755, Beispiel 1, bereitet war. Das bei der Umsetzung gebildete Gasgemisch (11) enthielt stündlich im wesentlichen 84,4 kg Stickstoff, 14,4 kg Sauerstoff, 22,9 kg Wasser, 9,0 kg Ammoniak, 1,1 kg 3-Methylpyridin, 9,9 kg 3-Cyanpyridin und 4,5 kg Kohlendioxid.

In dem Gaswäscher (10) der ersten Stufe wurde das Gasgemisch (11) mit stündlich 129,5 kg der Lösung (12) aus (20) bei 50 °C gewaschen. Die Lösung enthielt stündlich 9,9 kg Ammoniak und 4,6 kg Kohlendioxid. Aus dem Gaswäscher (10) fielen stündlich 165,7 kg der Lösung (13) an. Sie enthielt stündlich 12,3 kg Ammoniak, 1,1 kg 3-Methylpyridin, 9,9 kg 3-Cyanpyridin und 8,9 kg Kohlendioxid.

In (15) wurde diese Lösung mit stündlich 93,1 kg Benzol (14) extrahiert. Der Extrakt (16) enthielt stündlich 9,8 kg 3-Cyanpyridin, entsprechend einer Ausbeute von 99 %, bezogen auf das mit dem Gasgemisch (11) zugeführte 3-Cyanpyridin, und außerdem 1,5 kg 3-Methylpyridin. Der Extrakt wurde zur Gewinnung des 3-Cyanpyridins und 3-Methylpyridins sowie zur Rückgewinnung des Benzols fraktioniert destilliert. Das Benzol wurde in die Extraktion zurückgeführt und mit stündlich 0,4 kg frischem Benzol ergänzt.

Aus der wäßrigen Phase (17) der Extraktion (15) wurde in der Desorption (20) bei 8 bar und 145 °C Kohlendioxid abgetrieben. Das Abgas (21) enthielt stündlich 3,3 kg Kohlendioxid und außerdem 0,3 kg Ammoniak, 1,9 kg Wasser, 0,1 kg 3-Cyanpyridin und 0,4 kg Benzol. In die Desorption (20) wurden stündlich 7,9 kg Wasser (18) aufgegeben. Die Restlösung (12), die aus der Desorption (20) ablief, enthielt stündlich auf 139,5 kg Wasser 12,0 kg Ammoniak und 5,6 kg Kohlendioxid. Von ihr wurde ein Anteil von 82,5 % als Waschflüssigkeit in den ersten Gaswäscher (10) aufgegeben, der übrige Anteil von 17,5 % der Desorption (35) zugeführt (33).

Der zweite Gaswäscher (30) wurde bei 35 °C betrieben. In diesen wurde das Restgas (31) aus dem ersten Gaswäscher (10) eingeleitet, das stündlich 6,6 kg Ammoniak neben 0,2 kg Kohlendioxid enthielt. Dieses Gas wurde in (30) mit stündlich 96,0 kg in der Desorption (35) von Ammoniak befreitem Wasser (36) gewaschen.

Das Abgas (34) aus dem Gaswäscher (30) enthielt stündlich 0,6 kg Ammoniak. Es wurde zusammen mit dem Abgas (21) aus (20) einer Verbrennung zugeführt.

Die Waschflüssigkeit (32) aus dem zweiten Gaswäscher (30) enthielt stündlich 6,0 kg Ammoniak und 0,2 kg Kohlendioxid. Sie wurde der Desorption (35) zugeleitet und hier bei 2,2 bar und 108 °C vom Ammoniak befreit. Die Flüssigkeit (36), die aus der Desorption abgezogen wurde, war so gut wie frei von Ammoniak und Kohlendioxid. Von ihr wurde stündlich ein Anteil von 4,9 kg als Abwasser (38) verworfen, ein anderer Anteil (18) auf die Desorption (20) aufgegeben und der Rest als Waschflüssigkeit dem zweiten Gaswäscher (30) zugeführt. Das Gas (37), das aus der Desorption (35) anfiel, enthielt stündlich 12,6 kg Wasser, 8,1 kg Ammoniak und 1,2 kg Kohlendioxid. Es wurde in den Reaktor zurückgeführt.

**Patentansprüche**

1. Verfahren zur Gewinnung von 3-Cyanpyridin aus den bei der katalytischen Umsetzung von 3-Methylpyridin mit Ammoniak und Sauerstoff anfallenden Gasgemischen durch deren Behandlung mit Wasser, dadurch gekennzeichnet, daß man das Gasgemisch in der ersten Stufe bei Temperaturen von 30 bis 60 °C mit einer wässrigen Lösung behandelt, die bei der betreffenden Temperatur weitgehend oder vollständig mit Ammoniak gesättigt ist, in der zweiten Stufe bei um 10 bis 30 °C niedrigen Temperaturen mit Wasser behandelt, das weitgehend oder vollständig von Ammoniak frei ist, und die in der ersten Stufe anfallende Lösung mit einem organischen Lösungsmittel extrahiert.

2. Verfahren nach Anspruch 1, dadurch gekennzeichnet, daß man die Menge des Wassers so bemißt, daß in der ersten Stufe je Mol Kohlendioxid in dem zu behandelnden Gasgemisch mindestens 3 Mol Ammoniak und je Mol Ammoniak 0,1 bis 0,5 Liter Wasser vorhanden sind, und daß in der zweiten Stufe je Mol Ammoniak in dem Gasgemisch mindestens 0,2 Liter Wasser vorhanden sind.

3. Verfahren gemäß den Ansprüchen 1 und 2, dadurch gekennzeichnet, daß in der ersten Stufe je Mol Kohlendioxid in dem zu behandelnden Gasgemisch mindestens 5 Mol Ammoniak vorhanden sind.

4. Verfahren gemäß den Ansprüchen 1 bis 3, dadurch gekennzeichnet, daß die Gasgemische mit in Kreisläufen geführten wäßrigen Lösungen behandelt werden.

**Claims**

1. Process for the recovery of 3-cyanopyridine from the gas mixtures occuring in the catalytic reaction of 3-methyl pyridine with ammonia and oxygen, by treatment thereof with water, characterised in that the gas mixture is treated in a first stage at temperatures of from 30 to 60 °C with an aqueous solution which at the relevant temperature is substantially or completely saturated with ammonia, the gas mixture is treated in a second stage at temperatures from 10 to 30 °C lower with water which is substantially or completely free from ammonia, and the solution occuring in the first stage is extracted with an organic solvent.

2. A process according to claim 1, characterised in that the quantity of water is adjusted such that in the first stage, at least 3 mol of ammonia are present per mol of carbon dioxide in the gas mixture to be treated and from 0.1 to 0.5 litres of water are present per mol of ammonia, and that in the second stage, at least 0,2 litres of water are present per mol of ammonia in the gas mixture.

3. A process according to claims 1 and 2, characterised in that in the first stage, at least 5 mol of ammonia are present per mol of carbon dioxide in the gas mixture to be treated.

4. A process according to claims 1 to 3, characterised in that the gas mixtures are treated with a re-circulating aqueous solution.

## Revendications

1. Procédé pour l'obtention de 3-cyanopyridine à partir des mélanges gazeux formés lors de la transformation catalytique de 3-méthylpyridine avec de l'ammoniac et de l'oxygène, par le traitement de ceux-ci avec de l'eau, caractérisé en ce que l'on traite le mélange gazeux dans le premier stade à des températures de 30 à 60 °C avec une solution aqueuse qui est largement ou complètement saturée d'ammoniac à la température concernée, et dans le deuxième stade à des températures inférieures de 10 à 30 °C, avec de l'eau qui est dans une large mesure ou entièrement exempte d'ammoniac, et on extrait la solution formée dans le premier stade avec un solvant organique.

2. Procédé selon la revendication 1, caractérisé en ce que l'on choisit la quantité de l'eau de manière telle que dans le premier stade, il y ait au moins 3 moles d'ammoniac par mole d'anhydride carbonique dans le mélange gazeux à traiter et 0,1 à 0,5 l d'eau par mole d'ammoniac, et que dans le deuxième stade, il y ait au moins 0,2 l d'eau par mole d'ammoniac dans le mélange gazeux.

3. Procédé selon les revendications 1 et 2, caractérisé en ce que dans le premier stade, il y a au moins 5 moles d'ammoniac par mole d'anhydride carbonique dans le mélange gazeux à traiter.

4. Procédé selon les revendications 1 à 3, caractérisé en ce que les mélanges gazeux sont traités avec des solutions aqueuses recyclées.